# EUROPÄISCHE PATENTSCHRIFT

(11) **EP 2 756 815 B1**
(45) Veröffentlichungstag und Bekanntmachung des Hinweises auf die Patenterteilung: **09.09.2015**
(21) Anmeldenummer: 14151805.0
(22) Anmeldetag: 20.01.2014
(51) Int. Cl.: A61B 17/88, A61B 17/064, A61B 17/80

(54) **Sternum-Osteosynthese-System**
Sternum osteosynthesis system
Système d'ostéosynthèse du sternum

(30) Priorität: 22.01.2013 DE 102013000972; 05.03.2013 DE 102013102178
(43) Veröffentlichungstag der Anmeldung: 23.07.2014
(73) Patentinhaber: MedXpert GmbH, 79727 Eschbach (DE)
(72) Erfinder: Reisberg, Erhard, 79427 Eschbach (DE)
(74) Vertreter: Lermer, Christoph

(56) Entgegenhaltungen:
- WO-A1-2009/150047
- WO-A1-2010/004602
- CN-U- 202 235 649
- DE-A1-102006 042 277
- GB-A- 2 471 855
- US-A- 4 201 215

## Beschreibung

### TECHNISCHES GEBIET

Die vorliegende Anmeldung betrifft eine Implantatkomponente und ein Biegewerkzeug zur Sternum-Osteosynthese, umfassend eine Klammer mit einem ersten Schenkel und einem zweiten Schenkel, wobei der erste Schenkel und der zweite Schenkel über einen Verbindungsbereich verbunden sind. Außerdem betrifft die Erfindung Implantate zur Sternum-Osteosynthese, ein Implantatsystem zur Sternum-Osteosynthese und eine Reihe von Implantat-Auswahl- und Bearbeitungsinstrumenten sowie Biege-, Verform- und Anforminstrumenten zur Bearbeitung der Implantatkomponenten und Implantate.

### STAND DER TECHNIK

Der Begriff "Sternotomie" steht für die Öffnung des Brustkorbes (Thorax) im Bereich des Brustbeins (Sternum) zur Schaffung eines chirurgischen Zugangs zum Beispiel für Eingriffe am "offenen Herzen".

Die partielle oder totale Sternotomie wird als Zugangsweg für Operationen an den Organen des Mediastinums (Mittelfell) eingesetzt. Beispiele sind Operationen am offenen Herzen, Anlage von aortokoronaren Bypässen, Herztransplantationen, Tumoren der Thymusdrüse (sehr selten). In Ausnahmefällen ist die partielle Sternotomie auch zur Operation sehr großer retrosternaler Strumen erforderlich (Strumaresektion, Thyreoidektomie).

Es wird ein Längsschnitt über dem Brustbein durchgeführt. Dabei werden Haut und Unterhautfettgewebe durchtrennt. Eine lokale Blutstillung erfolgt, mittels Elektrokauter wird bis auf die Knochenhaut des Sternums präpariert. Von oben (Jugulum) und von unten (Processus xiphoideus) wird das Sternum mit dem Finger stumpf unterfahren und das lockere Bindegewebe an der Rückseite dadurch abgedrängt. Mit einer oszillierenden Säge oder einer speziellen Stichsäge wird das Sternum mittig längs getrennt. Der Brustkorb kann dann nahezu beliebig breit mittels eines Spreizers eröffnet werden.

Nach Abschluss des Eingriffs an den Weichteilen im Brustkorb muss das Sternum wieder verschlossen werden, wofür überwiegend Drähte aus Implantatstahl Verwendung finden. Im Allgemeinen erfolgt der Verschluss des Sternums mittels kräftiger, durch die Zwischenrippenräume geführter Drahtschlingen ("Drahtcerclagen").

Es sind verschiedene Materialstärken an Drähten verfügbar und auch die Technik der "Verdrahtung" selbst bietet diverse Möglichkeiten (Einzelnähte, 8er-Ligaturen, etc.).

Es gibt eine Vielzahl von Faktoren, die beim Einsatz von Drähten zu schwerwiegenden Komplikationen führen können. Bereits intraoperativ können durch die Führungsnadel am Draht kaum erkennbare Verletzungen von Blutgefäßen verursacht werden, die in einem Revisionseingriff behandelt werden müssen. Versagt beispielsweise die Drahtnaht zu einem frühen Zeitpunkt nach dem Eingriff, bevor der biologische Prozess der Osteosynthese (Heilung des getrennten Knochens) des Sternums weit genug fortgeschritten ist, "driften" die beiden Sternumhälften wieder auseinander oder reiben aneinander. Dies hat häufig schwerwiegende Folgen für den Patienten:
Der Patient muss sich zwecks Sekundärstabilisierung des Sternums einem zweiten chirurgischen Eingriff unterziehen. Abgesehen vom Operationsrisiko durch den Zweiteingriff an sich erhöht sich zwangsläufig das Risiko einer Entzündung oder Infektion, der Klinikaufenthalt sowie die gesamte Heilungsphase verlängert sich erheblich.

Die Konsequenzen einer tiefen postoperativen sternalen Infektion sind häufig fatal. Die Sterblichkeit liegt bei 15 - 50 % der betroffenen Patienten, nicht nur bedingt durch die Infektion an sich, sondern auch wegen Rupturen (Zerreißungen) des rechten Herzventrikels.

Bei Verwendung von Draht treten außerdem folgende Nachteile auf:
In Abhängigkeit vom verwendeten Material bzw. dessen Durchmesser liegt die maximale Zugbelastung von Draht bei ca. 20-22 kg, bis der Materialbruch eintritt. Demgegenüber ist aus der Literatur bekannt, dass ein spontaner Hustenanfall einen Druck von ca. 150 kg im Brustraum erzeugt. Demzufolge sind mindestens sechs, besser bis zu acht Drahtligaturen zur Stabilisierung einer Sternotomie zu verwenden, um diesem Druck widerstehen zu können. Bereits bevor es zum Materialbruch kommt, "dehnt" sich der Draht bei Überlastung in der Länge - Nähte können "aufgehen" bzw. es kann sich ein erweiterter Spalt zwischen den Sternumhälften bilden, der nicht zuwächst, wodurch häufig eine Instabilität des vorderen Brustkorbes erzeugt wird, und zu einer postoperativen Wundheilungsstörung, Entzündung oder Wundinfektion führen kann.

Beim Sternumverschluss werden die beiden losen Drahtenden mit einem Nadelhalter oder ähnlichen chirurgischen Instrument gefasst und umeinander verwunden. Häufig werden mehr als 4-5 Rotationsbewegungen der gefassten Drahtenden durchgeführt, wodurch der Draht an sich bereits geschwächt bzw. überdehnt wird - eine "Sollbruchstelle" ergibt sich, die innerhalb weniger Stunden, Tage oder Wochen zum Materialbruch und damit zum Lösen der Fixierung führt.

Bei Patienten mit eingeschränkter Knochenqualität (z. B. Osteoporose) führt die geringe Auflagefläche des Drahtes auf dem Knochen zu Drucknekrosen, der darunter liegende Knochen stirbt ab. Der Draht "schneidet ein" oder frakturiert den Knochen, was zu einer "Lockerung" der Fixation der Sternumhälften mit den beschriebenen Folgen führen kann.

Chirurgischer Draht besteht in der Regel aus Implantat-Stahl (Legierung aus Chrom-Nickel-Molybdän), der bei Allergikern zu massiven Fremdkörperreaktionen führen kann. Somit kann sich selbst bei unbeeinträchtigter Stabilisierung des Sternums durch eine Reaktion, ausgelöst durch das Fremdmaterial, eine Entzündung oder infektionsähnliche Reaktion des Gewebes ergeben.

Dokument GB2471855 A offenbart eine Implantatkomponente und ein Biegewerkzeug zur Sternum-Osteosynthese.

### AUFGABE DER ERFINDUNG

Die Aufgabe der vorliegenden Erfindung besteht darin, ein Implantat zur Sternum-Osteosynthese bereitzustellen, das eine längerfristige und verlässliche Stabilisierung des osteotomierten Sternums gewährleistet. Außerdem sollen die Implantate durch geeignete Werkzeuge bearbeitbar sein.

### TECHNISCHE LÖSUNG

Diese Aufgabe wird gelöst durch die Bereitstellung einer Implantatkomponente und eines Biegewerkzeugs gemäß dem Anspruch 1.

Die erfindungsgemäße Implantatkomponente, die selbst ein vollständiges Implantat sein oder eine Komponente eines Implantats mit weiteren Elementen sein kann, umfasst eine Klammer mit einem ersten Schenkel und einem zweiten Schenkel, wobei der erste Schenkel und der zweite Schenkel über einen Verbindungsbereich verbunden sind. Am Verbindungsbereich ist ein Element zum Eingriff mit einem kompatiblen Positionierelement eines Biegewerkzeugs bzw. zum positionsstabilen Abstützen eines kompatiblen Positionierelements eines Biegewerkzeugs angeordnet. Das Element selbst kann auch als Positionierelement bezeichnet werden, das mit dem kompatiblen Positionierelement des Biegewerkzeugs zusammenwirkt, um dieses relativ zum Implantat bzw. zur Implantatkomponente positionsstabil anzuordnen. Die positionsstabile Anordnung bedeutet, dass das Positionierelement des Biegewerkzeugs gegenüber dem Positionierelement des Implantats bzw. der Implantatkomponente bzgl. der Bewegung in einer oder zwei Dimensionen festgelegt, d.h. nicht bewegbar ist. Es findet dazu ein formschlüssiger Eingriff statt, der die Bewegungsrichtungen und die relative Ausrichtung der Positionierelemente begrenzt.

Die Implantatkomponente weist also ein Element auf, das gegenseitig mit einem kompatiblen Positionierelement eines Biegewerkzeugs zusammenwirken bzw. mit diesem in Eingriff gebracht und/oder an diesen sicher bzw. positionsfest angesetzt werden kann. So kann beispielsweise ein Niederhalter oder ein stationäres (d.h. bei der Zangenbetätigung gegenüber den Klemmbacken ortsfest am Implantat positioniertes) Fixierelement an einer Biegezange vorgesehen sein, das in das Element zum gegenseitigen Eingriff eingreift und so sicher gegenüber Positionsänderungen entlang der Oberfläche des Sternums gesichert ist. Das stationäre Fixierelement ist somit formschlüssig zumindest in zwei Dimensionen fixiert, kann jedoch aus dem Eingriff durch Abheben (dritte Dimension) wieder gelöst werden.

Das Element kann jedoch auch zum positionsstabilen Abstützen eines kompatiblen Positionierelements eines Biegewerkzeugs eingesetzt werden. So kann eine erste Klemmbacke einer Zange mit zwei Klemmbacken am Element abgestützt werden, während die andere Klemmbacke betätigt wird. Die erste Klemmbacke ist während des Biegevorgangs stationär, d.h. sie ist gegenüber am Implantat ortsfest abgestützt. Positionsstabiles Abstützen bedeutet, dass die erste Klemmbacke der Zange so an dem Element anliegt, dass es zumindest gegenüber Bewegungen quer zur Bewegungsrichtung des bewegbaren Werkzeugteils festgelegt ist. Ein Entfernen aus dem Anlageeingriff ist nur entgegen der Bewegungsrichtung des bewegbaren Werkzeugteils möglich. Das kompatible Positionierelement kann in gewissem Maß mit dem Element zum Eingriff und zum Abstützen verhakt werden.

Die Implantatkomponente kann sowohl für Sekundäreingriffe als auch als Standard bei der Primärstabilisation des Sternums nach Sternotomie eingesetzt werden. Sie hat dreidimensionale Funktionsstabilität, ist variabel einsetzbar, man kommt mit wenigen Varianten aus, die Konstruktion der Implantatkomponente führt dazu, dass wenige Implantat-Varianten erforderlich sind, etc. Insbesondere ist eine einfache und zeitsparende Handhabung gewährleistet.

Vorzugsweise ist die Implantatkomponente (wie auch die im Folgenden beschriebenen Implantate) aus biokompatiblem Material hergestellt. So kann ein Zweiteingriff zur Materialentfernung vermieden werden. Als Material kommt beispielsweise Titan in Frage. Titan kann unbegrenzt lange im Körper verbleiben. Da durch den Werkstoff und die der Anatomie angepasste Geometrie der Klammer weder biologische noch kosmetische Gründe (Tastbarkeit der Klammer) eine spätere Metallentfernung nahe legen, kann ein Zweiteingriff entfallen.

Die Applikation an einem Sternum wird wie folgt durchgeführt:
Zunächst wird eine Klammer geeigneter Größe ausgewählt und über das Sternum geführt, wobei zunächst die Schenkel der Klammer nach unten weisen. Anschließend werden mit einem ersten Biegewerkzeug, einer Implantat-Verformzange, die Schenkel um etwa 45° nach innen unter das Sternum gebogen. Zuletzt werden mit Hilfe eines weiteren Werkzeugs, einer Implantat-Anformzange, die Enden der Schenkel zur Anlage an der Unterseite des Sternums gebogen.

Bei dem Verfahren wird ausgenützt, dass die Klammer nicht vollständig unter dem Sternum hindurchgeführt werden muss, sondern bereits ein geringfügiges Hintergreifen des Sternums genügt, um die Implantatkomponente sicher zu befestigen bzw. die erforderliche Stabilität zu gewährleisten. Dadurch wird die Invasivität des Eingriffs wesentlich verringert, wenn nicht gar minimiert. Das Verletzungsrisiko von Gewebe oder Gefäßen wird minimiert.

Die Implantatkomponente ist im Wesentlichen wie eine Klammer ausgebildet. Die Klammer umschließt das Sternum nicht vollständig (jedoch zu mehr als 50 %) und kann in allen Intercostal-Räumen (Rippenzwischenräumen) entlang der gesamten Länge des Sternums eingesetzt werden.

Falls, bedingt durch eine auftretende Komplikation oder einen späteren Notfall, eine rasche Wiedereröffnung des Brustbeines vorgenommen werden muss, kann jede Implantatkomponente mittels in jedem Krankenhaus verfügbaren Standard-Instrumenten (Raspatorium, Elevatorium, Kneifzangen, etc.) innerhalb kurzer Zeit abgehoben oder durchtrennt und das Sternum wieder eröffnet werden. Vorteilhaft ist, dass die Implantatkomponente notfalls auch in Kliniken rasch entfernt werden können, die nicht über ein implantatspezifisches Entnahmeinstrumentarium verfügen.

Das erfindungsgemäße Element zum Eingriff bzw. zum Abstützen eines kompatiblen Positionierelements eines Biegewerkzeugs ermöglicht es, die Implantatkomponente bzw. das Implantat mit Werkzeugen zu bearbeiten, die genau positioniert und ggf. die Klammer in Position halten müssen.

Das Element zum Eingriff bzw. Abstützen weist einen ersten Vorsprung auf, der sich von einer ersten Seite des Verbindungsbereichs seitlich erstreckt, und einen zweiten Vorsprung, der sich von der gegenüberliegenden Seite des Verbindungsbereichs seitlich erstreckt. Das Element zum Eingriff bzw. Abstützen darf den Aufbau der Implantatkomponente nicht wesentlich komplizieren und es dürfen keine konstruktionsbedingten Geometrien vorhanden sein, die den Patienten oder den Operateur behindern. Durch das Ausbilden seitlicher Vorsprünge, die dieselbe Stärke/Dicke haben wie der benachbarte Verbindungsbereich, sind keine Erhöhungen vorhanden, die ggf. sogar nach der Operation tastbar wären. Der Aufbau ist denkbar einfach, bei gleichzeitig effizienter Möglichkeit, die Implantatkomponente während der Operation zuverlässig zu bearbeiten.

Die Vorsprünge können beidseitig des Verbindungsbereichs symmetrisch angeordnet sein. Sie können zusammen in etwa eine Schmetterlingsform aufweisen. D.h. ihre Abmessungen können so sein, dass sich die Vorsprünge nach außen hin, also jeweils vom Verbindungsbereich weg, verbreitern. Somit bilden sich hinter den Abschlusskanten der Vorsprünge Hinterschneidungen aus.

Die Vorsprünge schaffen insbesondere vier "Einhakstellen", die als Ansatzpunkt für einen Niederhalter einer Vorverformzange dienen. Da eine Klammer, deren Schenkel mit einer Zange zusammengedrückt werden, abhebt, ist der stationäre Niederhalter vorgesehen, der in das Element zum Eingriff bzw. Abstützen eingreift. Die Schenkel können so um ca. 45° nach innen gebogen werden.

Anschließend wird die Feinanformung mittels einer Zange mit zwei unterschiedlich langen Maulteilen/Klemmbacken durchgeführt. Das Element zum Eingriff bzw. Abstützen dient hier als Verankerungspunkte für das kurze Maulteil einer kompatiblen Rippenklammer-Fixierzange. Damit die Führungsnasen am Maulteilende sich gut einhaken können, ist der Winkel zwischen Vorsprung und Verbindungsbereich spitz, also kleiner als 90°. Damit können die Führungsnasen sicher am Implantat angesetzt werden.

Am Vorsprung sind kleine Radien am Übergang zum axialen Teil erforderlich, ebenso an der langen Basis der Raute oben und unten.

Insbesondere weisen der erste Vorsprung und/oder der zweite Vorsprung eine erste Stirnkante bzw. eine zweite Stirnkante und zwei Seitenkanten auf, wobei die Seitenkanten des Vorsprungs bzw. der Vorsprünge mit der jeweiligen Seitenkante des Verbindungsbereichs Winkel zum Eingriff des Positionierelements des Biegewerkzeugs und zur Anlage an den jeweiligen Kanten bilden.

Die Seitenkanten können mit der jeweiligen Kante des Verbindungsbereichs einen spitzen Winkel einschließen. Die Form der Vorsprünge ist dadurch jeweils in etwa ein Trapez, wobei die Stirnkanten länger sind als die Verbindungslinie zwischen dem Verbindungsbereich und dem jeweiligen Vorsprung. Dadurch verlaufen die nicht parallelen Seiten (Seitenkanten) des Trapezes in einem spitzen Winkel relativ zur Kante des Verbindungsstegs nach außen. Die Seitenkanten können leicht gekrümmt sein und schließen jedenfalls mit den Kanten des Verbindungsbereichs einen Winkel kleiner 90° ein, sodass das Biegewerkzeug sicher positioniert werden kann.

Der Winkel kann insbesondere größer als 60°, insbesondere größer als 80°, und kleiner als 90°, insbesondere kleiner als 85° sein. Der Winkel wird häufig etwa zwischen 80°und 85° liegen.

Die erste Stirnkante und/oder die zweite Stirnkante können gekrümmt ausgebildet sein. insbesondere sind sie nach außen hin konvex gekrümmt.

Die Oberfläche des Elements zum Eingriff bzw. Abstützen ist insbesondere bündig mit dem Verbindungsbereich der Klammer ausgebildet. Dies wird durch die Konstruktion der Ausbildung seitlicher Vorsprünge als Element zum Eingriff bzw. Abstützen ermöglicht. Es ist somit nicht nötig, an der Oberseite der Implantatkomponente irgendwelche Erhöhungen oder Vorsprünge auszubilden, die den Operateur und den Patienten behindern können. Das Implantat bleibt somit weitgehend unbemerkt, auf eine Sekundäroperation zur Entfernung kann damit im Regelfall verzichtet werden.

Der erste Vorsprung und/oder der zweite Vorsprung weisen vorzugsweise dieselbe Dicke auf wie der Verbindungsbereich des Implantats.

Die Aufgabe wird auch gelöst durch ein Implantat zur Sternum-Osteosynthese, umfassend: eine erste Implantatkomponente wie oben beschrieben, und eine zweite Implantatkomponente wie oben beschrieben, einen Verbindungssteg, der die erste Implantatkomponente und die zweite Implantatkomponente verbindet. Der Verbindungssteg weist wenigstens eines, vorzugsweise mehrere nebeneinander entlang einer Achse angeordnete Stegelemente auf, wobei das bzw. die Stegelemente jeweils einen Rahmen aufweisen, der eine Öffnung begrenzt. Insbesondere ist der Verbindungssteg längenveränderbar, wie im folgenden beschrieben wird.

Der Verbindungs- oder Mittelsteg des Implantats sollte insbesondere längenveränderbar sein. Der Mittelsteg bezweckt, dass sich ein gespaltenes Sternum nicht in der Längsachse verschieben kann und dient zur Versorgung von Schrägfrakturen des Sternums zum Beispiel in Folge von Autounfällen, bei welchen beispielsweise der Sicherheitsgurt besagte Schrägfrakturen verursacht hat. Der Mittelsteg sollte zudem dreidimensional verformbar sein, notfalls also torquierbar bzw. über die Fläche auf- und abbiegbar. Dies ist durch die Struktur des Verbindungsstegs der vorliegenden Erfindung realisiert.

Der bzw. die Rahmen des bzw. der Stegelemente begrenzen eine in etwa rechteckige oder rautenförmige Öffnung, wobei der bzw. die Rahmen so angeordnet sind, dass eine Diagonale der rechteckigen bzw. rautenförmigen Öffnung entlang der zentralen Achse, die sich senkrecht zu den Klammern entlang des Verbindungsstegs erstreckt, auf der Achse liegt. Dadurch wird eine Verformbarkeit in Form einer Verlängerung und Verkürzung des Stegs ermöglicht.

Vorzugsweise weist der Verbindungssteg zwei äußere Stegelemente und eines oder mehrere benachbart in Reihe angeordnete innere Stegelemente auf, wobei die beiden äußeren Stegelemente jeweils an einer Stirnkante des Vorsprungs der ersten Implantatkomponente bzw. der zweiten Implantatkomponente angeordnet sind.

Der Verbindungssteg weist insbesondere die gleiche Dicke auf wie die Klammer bzw. das Element zum Eingriff bzw. Abstützen eines kompatiblen Positionierelements eines Biegewerkzeugs.

Die Klammer der ersten Implantatkomponente kann eine andere Spannweite aufweisen als die Klammer der zweiten Implantatkomponente.

Dieses Implantat kann prinzipiell als eine Doppel-T-Klammer gefertigt und dann gebogen werden. Es können nun Varianten mit gleichen (symmetrische T-Form) und unterschiedlichen (asymmetrische T-Form) Klammergrößenverhältnissen zwischen der ersten und der zweiten Implantatkomponente bereitgestellt werden. Die Varianten können durch einen Farbcode markiert werden.

Ein weiteres erfindungsgemäßes Implantat umfasst: wenigstens eine Implantatkomponente wie oben beschrieben, und eine Befestigungseinrichtung zur Befestigung eines stabförmigen Verbindungselements an der Befestigungseinrichtung, wobei die Befestigungseinrichtung am Element zum Eingriff bzw. Abstützen befestigt ist.

Auch bei diesem Modell muss für die Fixierzange eine möglichst verlässliche "Einhakstelle" vorgesehen werden. Dies wird durch den Einsatz der erfindungsgemäßen Implantatkomponente im Aufbau gewährleistet.

Die Befestigungseinrichtung kann einen Grundkörper mit einer Auflagefläche und seitlich der Auflagefläche angeordnete Führungselemente aufweisen, wobei die Führungselemente jeweils einen Steg und einen sich von der Oberkante des Stegs nach innen erstreckenden Führungsvorsprung aufweisen, die mit einem Seitenbereich der Auflagefläche jeweils im Querschnitt U-förmige Führungsaufnahmen begrenzen.

Die Führungsvorsprünge der Führungselemente weisen vorzugsweise eine Eingriffsstruktur, insbesondere eine Zahnstruktur, auf.

Ein erfindungsgemäßes Implantatsystem umfasst: wenigstens ein Implantat wie oben beschrieben, und ein stabförmiges Verbindungselement zum Einschub in die Führung der Befestigungseinrichtung und zur Befestigung an der Führung. Es können bespielsweise zwei Implantate mit jeweils einer Führung vorgesehen sein.

Das stabförmige Verbindungselement weist insbesondere einen Befestigungsbereich mit einer Eingriffsstruktur, insbesondere einer Zahnstruktur, auf, die zur Befestigung des stabförmigen Verbindungselements an der Führung mit der Zahnstruktur der Führungsstege im Eingriff bringbar ist.

In sämtlichen Ausführungsformen mit zwei oder mehreren Klammern kann die Anzahl der Klammern variiert werden. Außerdem können eine oder mehrere der Klammern mit nur einem Zinken ausgebildet sein. Insbesondere können entlang eines Verbindungsstegs die Zinken versetzt zueinander angeordnet sein und unterschiedliche Breite aufweisen.

Ein erfindungsgemäßes Biegewerkzeug umfasst zwei Klemmbacken und ein Positionierelement zum Eingriff bzw. zum Abstützen des Biegewerkzeugs an einer Implantatkomponente wie oben beschrieben bzw. an einer Implantatkomponente eines Implantats wie oben beschrieben.

Das Positionierelement kann wenigstens zwei Vorsprünge, beispielsweise zwei oder vier Vorsprünge, zum Abstützen des Biegewerkzeugs am Element zum Eingriff bzw. Abstützen des Biegewerkzeugs aufweisen.

Das Positionierelement kann beispielsweise als Niederhalter zum Anpressen des Implantats an das Sternum während des durch zwei Klemmenbacken ausgeübten Klemmvorgangs ausgebildet sein. Eine Zange zum Vorverformen ist symmetrisch und hat einen Niederhalter.

Bei einer asymmetrischen Zange mit Positionierelement ist zu berücksichtigen, dass nicht sehr viel Raum zwischen den Rippen neben dem Sternum zur Einführung des umfassenden Maulteils existiert. Demzufolge darf die Zange nicht zu bauchig sein. Ziel ist, das längere Maulteil möglichst nahe über der Klammer um das Sternum herum nach unten führen zu können, während das kürzere Maulteil über das Positionierelement am Implantat in Position gehalten wird. Dies wird durch eine geeignete Geometrie erreicht. Mit Hilfe des Implantats und des Werkzeugs wird das Risiko von Verletzungen von Gefäßen und Gewebe seitlich des Sternums reduziert.

Ein weiteres Biegewerkzeug ist zum Verbiegen eines Verbindungsstücks eines Implantats wie oben beschrieben konzipiert. Das Biegewerkzeug weist zwei Klemmbacken auf, wobei wenigstens eine Klemmbacke einen Vorsprung zum Eingriff in eine im Verbindungssteg des Implantats ausgebildete Öffnung aufweist.

Ein weiteres Biegewerkzeug umfasst zwei Klemmbacken mit durch Betätigen des Biegewerkzeugs miteinander in Kontakt bringbaren Kanten, wobei an der Vorderseite der Klemmbacken jeweils eine sich von der jeweiligen Kante nach außen erstreckende Vertiefung ausgebildet ist, wobei die äußere Wand der Vertiefung eine Krümmung derart aufweist, dass ein Rahmen eines Stegelements eines Implantats wie oben beschrieben umgriffen und bei Betätigung des Biegewerkzeugs verformt werden kann.

Im Rahmen der Erfindung sollen auch alle Kombinationen von Implantaten und passenden Werkzeugen beansprucht werden.

So umfasst beispielsweise eine erfindungsgemäße Kombination eine Implantatkomponente oder ein Implantat wie oben beschrieben, und ein insbesondere zangenartiges Biegewerkzeug, wobei das Biegewerkzeug ein zum Element zum gegenseitigen Eingriff bzw. zum positionsstabilen Abstützen kompatibles Positionierelement aufweist. Das kompatible Positionierelement kann beispielsweise Vorsprünge aufweisen, wie oben beschrieben und in der nachfolgenden Figurenbeschreibung spezifiziert.

Ein erfindungsgemäßes Instrument zur Größenbestimmung eines einzusetzenden Implantats umfasst zwei gegeneinander drehbare Hebel, eine Messeinrichtung mit einem am ersten Hebel angeordneten Zeiger und einer am zweiten Hebel angeordneten Skala, wobei der Zeiger in Abhängigkeit vom Drehwinkel zwischen den Hebeln einen bestimmten Wert auf der Skala anzeigt.

Die Skala kann einen länglichen Abschnitt, beispielsweise als Metallstreifen ausgebildet, aufweisen, der nebeneinander angeordnete Felder aufweist, die unterschiedlichen Bereichen von Drehwinkeln zwischen den Hebeln entsprechen. Die Felder können farblich entsprechend den Implantatgrößen markiert sein. Der längliche Abschnitt kann in einem bestimmten Radius gebogen sein, der insbesondere dem Abstand zwischen dem länglichen Element und der Drehachse, die die Hebel verbindet, entspricht.

Mit Hilfe des Instruments ist es möglich, auf Schablonen zur Bestimmung der erforderlichen Implantatgrößen zu verzichten. Außerdem werden Implantate steril an den Operateur geliefert, sodass es nicht möglich ist, die richtige Größe hinsichtlich der Passgenauigkeit intraoperativ durch Ansetzen und Ausprobieren verschiedener Implantate zu ermitteln. Mit Hilfe des Messinstruments wird somit verhindert, dass passgenaue Implantate erst nach Versuchen intraoperativ ermittelt werden.

Die Skala des Messinstrumentes ist farbcodiert. Somit muss nicht irgendein metrischer Messwert ermittelt werden, welcher auf die Skala übertragen wird, sondern es kann ohne "Umrechnung" ein bestimmungsgemäßes Implantat durch die gleiche Farbe der Skala definiert werden. Das ist wesentlich sicherer und direkter, als eine "Messung" mit anschließender Zuordnung, wie z.B. über metrische Tabellen. Durch die Anzeige einer bestimmten Farbe auf der Skala wird unmittelbar die Farbe des einzusetzenden Implantats angezeigt.

### BESCHREIBUNG DER FIGUREN

Weitere Merkmale und Vorteile der Erfindung ergeben sich aus der nachfolgenden Beschreibung bevorzugter Ausführungsbeispiele anhand der Figuren. Es zeigen:
- Figur 1: eine erste Ausführungsform eines erfindungsgemäßen Implantats;
- Figur 2: einen Ausschnitt aus der Figur 1;
- Figur 3: eine zweite Ausführungsform des erfindungsgemäßen Implantats;
- Figur 4: eine dritte Ausführungsform des erfindungsgemäßen Implantats;
- Figur 5: eine vierte Ausführungsform des erfindungsgemäßen Implantats;
- Figur 6: eine fünfte Ausführungsform des erfindungsgemäßen Implantats;
- Figur 7: einen Verbindungsstab, der in einem System mit dem Implantat aus Figur 5 eingesetzt wird;
- Figur 8: eine schematische Darstellung unterschiedlicher Implantattypen eines Implantatsystems;
- Figur 9a: ein erstes Biegewerkzeug zur Verwendung in erfindungsgemäßen Implantatsystemen;
- Figur 9b: einen Ausschnitt aus der Figur 8a;
- Figur 10a: ein zweites Biegewerkzeug zur Verwendung in erfindungsgemäßen Implantatsystemen;
- Figur 10b: einen Ausschnitt aus der Figur 9a;
- Figur 11: ein drittes Biegewerkzeug zur Verwendung in erfindungsgemäßen Implantatsystemen;
- Figur 12a: ein viertes Biegewerkzeug zur Verwendung in erfindungsgemäßen Implantatsystemen;
- Figur 12b: einen Ausschnitt aus der Figur 12a;
- Figur 12c: eine andere Ausführungsform der Figur 12a; und
- Figur 13: ein erfindungsgemäßes Implantat-Auswahlgerät.

### DETAILLIERTE BESCHREIBUNG BEVORZUGTER AUSFÜHRUNGSBEISPIELE

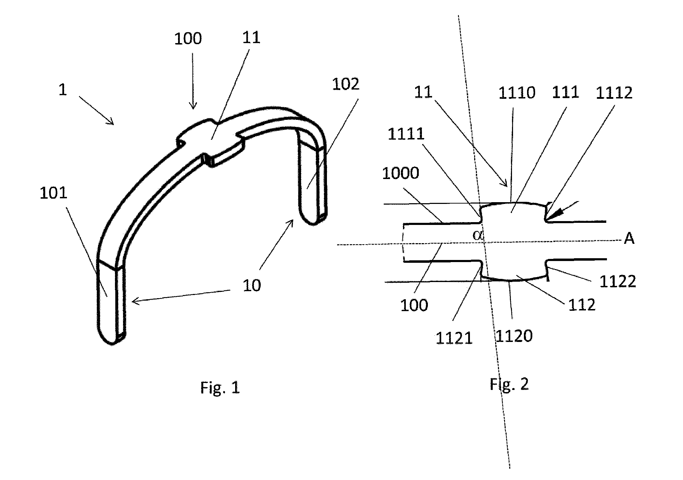

Die Figur 1 zeigt eine erste Ausführungsform eines erfindungsgemäßen Implantats 1 bzw. einer erfindungsgemäßen Implantatkomponente 1 zur Sternum-Osteosynthese. Das Implantat 1 weist eine Klammer 10 mit einem ersten Schenkel 101, einem zweiten Schenkel 102 und einem Verbindungsbereich 100 auf.

Im Verbindungsbereich 100 ist erfindungsgemäß ein Element 11 zum Eingriff bzw. zum positionsstabilen Abstützen eines kompatiblen Abstützelements eines Biegewerkzeugs (nicht dargestellt) ausgebildet. Vorzugsweise ist das Implantat 1 einstückig bzw. einteilig ausgebildet, beispielsweise aus Titan oder anderen biologisch neutralen bzw. bioinerten Materialien.

Das Element 11, das in der Figur 2 in einer Draufsicht vergrößert dargestellt ist, weist beidseitig des Verbindungsbereichs 100 der Klammer 10 Vorsprünge 111 bzw. 112 auf. An einer Seite erstreckt sich seitlich der erste Vorsprung 111, auf der anderen Seite der zweite Vorsprung 112. Die Vorsprünge 111 und 112 erstrecken sich im Wesentlichen senkrecht vom Verbindungsbereich 100 nach außen. Die Abschlusskanten 1110 bzw. 1120 verlaufen im Wesentlichen parallel zur Kante 1000 des Verbindungsbereichs 100, sind jedoch beidseitig mit großem Radius leicht nach außen hin gekrümmt ausgebildet. Die sich im Wesentlichen senkrecht nach außen erstreckenden Seitenkanten 1111, 1112 bzw. 1121, 1122 der Vorsprünge 111 bzw. 112 weisen jeweils eine Hinterschneidung auf, d.h. die Vorsprünge 111 und 112 verbreitern sich zu ihren Abschlusskanten 1110 bzw. 1120 hin. Der Winkel α zwischen der Längsachse A des Verbindungsbereichs 100 und den Seitenkanten 1111, 1112 bzw. 1121, 1122 beträgt etwas weniger als 90°, insbesondere zwischen 60°, 70° bzw. 80° und 85°. In den Hinterschneidungen kann ein Eingriffs- bzw. Abstützelement eines Biegewerkzeugs sicher angesetzt werden. Das Element 11 weist somit beidseitig des Verbindungsbereichs 100 der Klammer 10 Vorsprünge auf, die gemeinsam mit den Kanten des Verbindungsbereichs Winkel zum Eingriff des Positionierelements eines Biegewerkzeugs bilden.

Insgesamt weist das Element 11 zum Eingriff bzw. Abstützen eines Werkzeugs andeutungsweise eine Schmetterlingsform auf, da die Kanten 1111 und 1121 bzw. 1112 und 1122 jeweils in einem stumpfen Winkel 2*α zueinander verlaufen und die Kanten 1111 und 1112 bzw. 1121 und 1122 jeweils spiegelverkehrt und symmetrisch ausgebildet und angeordnet sind.

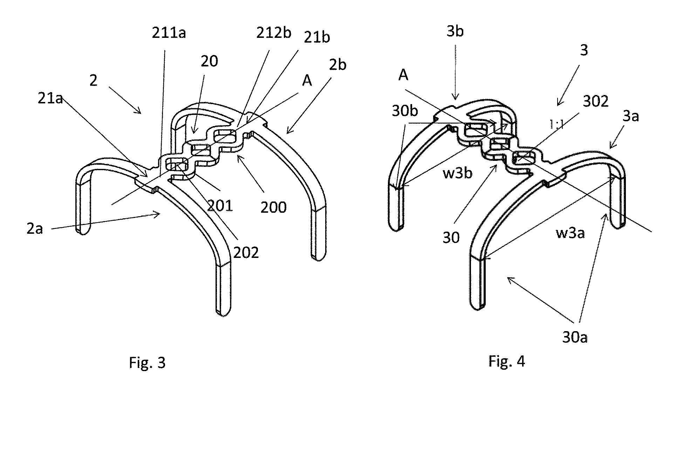

In der Figur 3 ist eine zweite Ausführungsform eines erfindungsgemäßen Implantats 2 dargestellt. Das Implantat 2 weist eine erste Implantatkomponente 2a und eine zweite Implantatkomponente 2b auf, die über einen Verbindungssteg 20 verbunden sind. Die Implantatkomponenten 2a und 2b haben jeweils im Wesentlichen denselben Aufbau wie das im Zusammenhang mit den Figuren 1 und 2 beschriebene Implantat bzw. die Implantatkomponente 1 gemäß der ersten Ausführungsform. Sich entsprechende Elemente sind mit der Zahl "2" statt "1" und einem Buchstaben bezeichnet. Aus diesem Grund wird der Aufbau der ersten Implantatkomponente 2a und der zweiten Implantatkomponente 2b nicht näher beschrieben.

Die erste Implantatkomponente 2a und die zweite Implantatkomponente 2b sind über den Verbindungssteg 20, der sich zwischen einem der Vorsprünge 211a des Elements 21a zum Eingriff bzw. zum Abstützen eines kompatiblen Abstütz- oder Positionierelements eines Biegewerkzeugs der ersten Implantatkomponente 2a und einem der Vorsprünge 212b des Elements 21b zum Eingriff bzw. zum Abstützen eines kompatiblen Abstützelements eines Biegewerkzeugs der zweiten Implantatkomponente 2b erstreckt.

Der Verbindungssteg 20 weist Stegelemente 200, in vorliegendem Fall drei Stegelemente 200, auf, die benachbart entlang einer Längsachse A des Verbindungsstegs 20 angeordnet sind. Jedes der Stegelemente 200 weist einen Rahmen 201 auf, der eine mittige Öffnung 202 begrenzt. Die Öffnung ist in etwa quadratisch mit abgerundeten Ecken ausgebildet. Die Stegelemente 200 sind so angeordnet, dass sich jeweils eine Diagonale entlang der Längsachse A erstreckt. Eine erste Ecke der Stegelemente 200 ist dementsprechend mit einem Vorsprung 211a, 212b oder mit einer Ecke eines benachbarten Stegelements 200 verbunden. Eine der ersten Ecke gegenüberliegende zweite Ecke der Stegelemente 200 ist mit einer Ecke eines benachbarten Stegelements 200 verbunden. D. h. eine Ecke der äußeren der Stegelemente 200 ist jeweils mit einem Vorsprung 211a, 212b verbunden, die andere mit der Ecke des benachbarten Stegelements 200. Gegenüberliegende Ecken der mittleren oder inneren Stegelemente 200 sind jeweils mit der Ecke eines benachbarten Stegelements 200 verbunden.

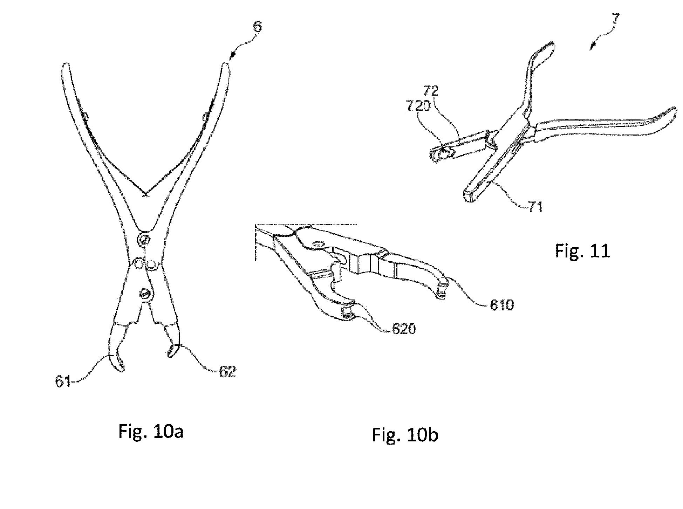

Die Öffnungen 202 der Stegelemente 200 sind zum Eingriff eines kompatiblen Eingriffsvorsprungs eines Biegewerkzeugs (Figur 11, 720) bestimmt und ausgebildet. Auf diese Weise kann das Biegewerkzeug die Stegelemente 200 sicher greifen, so dass der Steg 20 in verschiedener Weise gebogen werden kann, beispielsweise um die Achse A verdreht (torquiert) werden kann. Mit einem Werkzeug, das in die Öffnungen 202 der beiden äußeren Stegelemente 200 eingreift, kann der Steg 20 verkürzt bzw. gestaucht werden, indem die beiden äußeren Stegelemente 200 aufeinander zu gepresst werden, wodurch sich der Rahmen 201 des mittleren Stegelements 200 verbiegt und die Öffnung 202 des mittleren Elements 200 eine Art Rautenform annimmt, deren kürzere Diagonale entlang der Achse A angeordnet ist. Insbesondere sind nur die beiden äußeren Öffnungen 202 formschlüssig auf das Instrument aus Figur 11 bzw. die Form 720 abgestimmt. Die jeweils mittlere Öffnung ist immer kleiner, um ein unbewusstes Aufnehmen mit der Form 720 aus Figur 11 zu verhindern. Das zentrale Rautenelement 200 bleibt dadurch immer frei und es verbleibt ein großzügiger Abstand zwischen zwei Zangen, siehe Figur 11. Das zentrale Element 200 verhindert das Abscheren eines Steges beim Verdrehen in der Längsachse und erlaubt dem Material in der Bewegung zu "fließen".

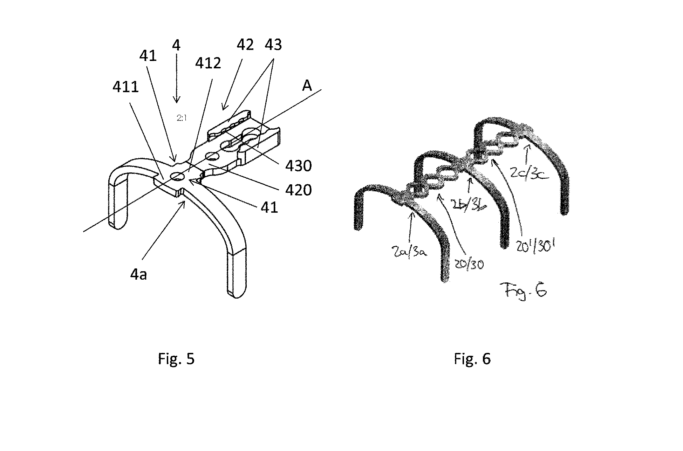

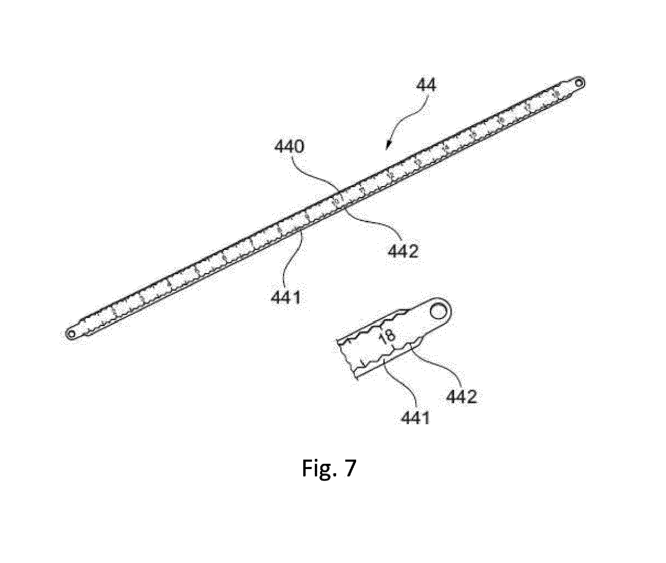

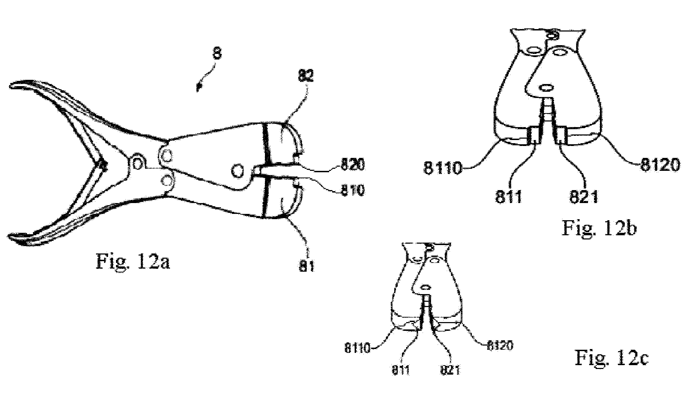

Die in der Figur 12 abgebildete Zange ist zum Zudrücken des Implantatschuhs 43 aus Figur 5 zur Schaffung einer Verbindung mit dem Steg 44 aus Figur 7 vorgesehen und hat deshalb parallel schließende Aussparungen 811, 821 mit Kanten 8110 bzw. 8120 gemäß Figur 12b.

Ein ähnliches Werkzeug gemäß Figur 12a weist Aussparungen 811, 821 mit hinteren Kanten 8110, 8120 mit einem Winkel auf, das zur Verlängerung eines rautenförmigen Steges 200 in Figur3 an den freien Ecken eines der quadratischen Rahmen 201 der Stegelemente 200 angreift. Der Steg 20 kann verlängert werden, indem die freien gegenüberliegenden Ecken des Rahmens 201 zusammengepresst werden. In diesem Fall nimmt die Öffnung 202 eine rautenförmige Form an, wobei die längere Diagonale der Raute entlang der Achse A angeordnet ist. Dieser Vorgang kann an einzelnen, mehreren oder allen Elementen 200 durchgeführt werden. In der Figur 4 ist eine Variante 3 des Implantats 2 aus der Figur 3 dargestellt. Das Implantat 3 dieser dritten Ausführungsform unterscheidet sich vom Implantat 2 der zweiten Ausführungsform lediglich dadurch, dass die Implantatkomponenten 3a und 3b, die im Wesentlichen der im Zusammenhang mit den Figuren 1 und 2 beschriebenen Implantatkomponente 1 entsprechen, im Gegensatz zur zweiten Ausführungsform Klammern 30a und 30b unterschiedlicher Geometrie aufweisen. Im vorliegenden Fall ist die Klammer 30a größer als die Klammer 30b, d.h. sie hat eine größere Spannweite w (w 3a > w3b). Auf diese Weise kann das Implantat 3 am Sternum fixiert werden, wobei die erste Implantatkomponente 3a an einem Bereich des Sternums mit größerem Umfang befestigt werden kann, und die zweite Implantatkomponente 3b an einem Bereich des Sternums mit kleinerem Umfang befestigt werden kann. Die Länge des Verbindungsstegs 30 kann durch entsprechende Biegewerkzeuge an den Abstand und die Geometrie des Sternums zwischen den Befestigungsbereichen der Implantatkomponenten 3a und 3b angepasst werden. In ähnlicher Weise kann die Lage der Implantatkomponenten 3a und 3b, beispielsweise der Grad, in dem diese zueinander um die Achse A gegeneinander verdreht sind, bzw. der Winkel zwischen den Ebenen, die die Klammern 30a und 30b der Implantatkomponenten 3a bzw. 3b aufspannen, festgelegt werden.

Das im Zusammenhang mit den Figuren 1 und 2 beschriebene Implantat bzw. Implantatkomponente 1 kann als "Single-Clip" bezeichnet werden. Die in Figuren 3 und 4 beschriebenen Implantate 2 und 3 bestehen demnach aus durch jeweils einen Verbindungssteg 20 bzw. 30 verbundene Single-Clips. 2a, 2b bzw. 3a, 3b.

In der Figur 5 ist eine vierte Ausführungsform eines erfindungsgemäßen Implantats 4 dargestellt. Das Implantat 4 besteht im Wesentlichen aus einer Implantatkomponente 4a, die vom Aufbau her einer Implantatkomponente 1 gemäß der ersten Ausführungsform entspricht. Die Implantatkomponente 4a ist daher nicht genauer beschrieben.

In der vierten Ausführungsform der Erfindung schließt sich an einen Vorsprung 412 der beiden Vorsprünge 411, 412 des Elements 41 zum Eingriff bzw. Abstützen eines Biegewerkzeugs eine Halterung bzw. ein Befestigungseinrichtung 42 an, die einen Grundkörper 420 mit einer Auflagefläche für ein Verbindungselement 44 (vgl. Figur 7) aufweist. Außerdem weist die Halterung 42 beidseitig des Grundkörpers 420 sich von der Auflagefläche des Grundkörpers 420 nach oben erstreckende Seitenstege auf, die am oberen Ende jeweils einen sich nach innen erstreckenden Führungsvorsprung aufweisen. Die Stege und die Führungsvorsprünge bilden jeweils ein L-förmiges Führungselement 43, die zusammen eine Führung zum geführten Einschieben eines Verbindungselements 44 bilden. Durch die L-förmige Ausbildung bilden die Führungselemente 43 zusammen mit den Randbereichen der Auflagefläche des Grundkörpers 420 eine im Querschnitt senkrecht zur Achse A U-förmige Führungsaufnahme (beidseitig), in die das stabförmiges Verbindungselement 44 in Richtung der Achse A geführt eingeschoben werden kann.

Außerdem weisen die sich von den Stegen erstreckenden Führungsvorsprünge an den sich gegenüber liegenden Kanten jeweils eine Zahnstruktur 430 auf.

Die Figur 6 zeigt eine fünfte Ausführungsform des erfindungsgemäßen Implantats. Diese Ausführungsform entspricht im Wesentlichen den Ausführungsformen gemäß den Figuren 3 und 4 mit dem Unterschied, dass neben der ersten Implantatkomponente 2a bzw. 3a und der zweiten Implantatkomponente 2b bzw. 3b, die durch Verbindungsstege 20 bzw. 30 verbunden sind, eine dritte Implantatkomponente 2c/3c vorgesehen ist, die über einen weiteren Verbindungssteg 20'/30' mit der zweiten Implantatkomponente 2b/3b verbunden ist. Alle drei Implantatkomponenten 2a/3a, 2b/3b, 2c/3c entsprechen sich hinsichtlich des Aufbaus. Die Spannweite der Klammern der drei Implantatkomponenten 2a/3a, 2b/3b, 2c/3c kann gleich groß oder unterschiedlich groß sein.

In der Figur 7 ist ein Verbindungselement 44 dargestellt, das in die Führung 43 eingeschoben werden kann. Das Verbindungselement 44 weist einen stabförmigen Körper 440 auf, der beidseitig eine Aussparung 441 aufweist. Diese sollten wenigstens im End- bzw. Verbindungsbereich vorgesehen sein. Die Aussparung 441 wird durch eine waagerechte und eine senkrechte Wand begrenzt. Die senkrechte Wand weist eine Zahnstruktur 442 auf, die komplementär zur Zahnstruktur 430 der Führungsvorsprünge der Führungselemente 43 ausgebildet ist. Während die sich beidseitig nach außen erstreckenden waagerechten Wände in die U-förmigen Aufnahmen der Führungselemente 43 eingeführt werden, kann das stabförmige Verbindungselement 44 am Implantat 4 ausgerichtet und durch Zusammendrücken der Stege der Führung 43 arretiert werden, wobei die Verzahnung 430 der Führung 43 und die Verzahnung 442 des Verbindungselements 44 in gegenseitigen Eingriff gebracht werden. Der Stab 44 weist jedoch vorzugsweise beidseitig über seine gesamte Länge eine Aussparung 441 und eine Zahnstruktur 442 auf, wie in der Figur 7 dargestellt. Auf diese Weise ist es möglich, eine Vielzahl von Implantaten 4, mindestens aber von zwei Implantaten 4, auf den Stab "aufzufädeln". D.h. die Implantate 4 sind entlang der Längsachse des Stabs 44 beabstandet aufgereiht. Die Abstände der aufgereihten Implantate 4 orientieren sich an den Abständen der Interkostalräume in axialer Sternumausrichtung. Eine Längenvariabilität entsprechend erforderlicher Abstände aufgrund der gegebenen Anatomie ist möglich. Die Führungen bzw. Schuhteile 43 der äußeren Implantate sind gegeneinander ausgerichtet, je nach Platzverhältnissen zwischen den Intercostal-Räumen können diese jedoch auch in Reihe liegend ausgerichtet sein. Sofern eines oder mehrere mittlere Implantate 4 vorgesehen sind, kann die Führung 43 relativ zum Element 41 zum Eingriff, Abstützen bzw. Positionieren eines Biegewerkzeugs in entgegengesetzte Richtungen, d.h. zu entgegengesetzten Enden des Stabs 44, weisen. Die Ausrichtung des Implantats 4 richtet sich nach den anatomischen Notwendigkeiten.

Der andere End- bzw. Verbindungsbereich des stabförmigen Verbindungselements 44 wird in der Regel mit einem weiteren Implantat 4 gemäß der vierten Ausführungsform aus der Figur 5 verbunden. Bei dieser Konstruktion können beidseitig die Position und der Einschubweg des stabförmigen Verbindungselements 44 in die Führungen 43 der Implantate 4 variiert, eingestellt und fixiert werden. Dadurch kann die Länge der Verbindung zwischen zwei Implantaten 4 je nach Bedarf eingestellt werden. Es ist denkbar, dass entlang des stabförmigen Verbindungselements 44 an beliebiger Stelle, also beispielsweise auch in etwa mittig, Implantate 4 angeordnet und befestigt werden.

Im Verbindungsbereich zwischen dem Vorsprung 412 und dem Halteelement 42 ist im vorliegenden Ausführungsbeispiel ein verschmälerter Bereich vorgesehen, der verbiegbar und/oder verdrehbar ausgebildet sein kann. Die beidseitig dieses Bereichs angeordneten kreisförmigen Bohrungen sind zum Eingriff eines Biegewerkzeugs (z.B. Dreipunkt-Zange) bestimmt. Auf den Verbindungsbereich kann jedoch auch verzichtet werden, d.h. die Führung 43 kann sich unmittelbar an den Vorsprung 412 anschließen.

Das im Zusammenhang mit den Figuren 1 und 2 beschriebene Implantat bzw. die Implantatkomponente 1 kann als "Single-Clip" bezeichnet werden. Die in Figuren 3 und 4 beschriebenen Implantate 2 und 3 bestehen demnach aus durch jeweils einen Verbindungssteg 20 bzw. 30 verbundene Single-Clips. 2a, 2b bzw. 3a, 3b. Das in der Figur 6 beschriebene Implantat 4 besteht aus einem Single-Clip 4a und einer daran befestigten Halterung 42.

In einem Implantatsystem aus mehreren Implantaten und ggf. Werkzeugen können Single-Clips gemäß der Figur 1 in verschiedenen Größen, z.B. mit unterschiedlicher Spannweite der Klammern, bereitgestellt werden. Implantate gemäß den Figuren 3, 4,5 oder 6 können ebenfalls mit Single-Clips unterschiedlicher Klammergröße bereitgestellt werden. Im Implantat 2 gemäß der zweiten Ausführungsform sind die Single-Clips 2a und 2b im Wesentlichen identisch, d.h. sie haben die gleiche Größe. In der dritten Ausführungsform gemäß Figur 4 sind die beiden im Implantat 3 vorhandenen Single-Clips 3a und 3b mit unterschiedlicher Spannweite ausgestattet. In einem Implantatsystem können Implantate 3 gemäß der dritten Ausführungsform mit Klammern unterschiedlicher Größenverhältnisse und unterschiedlichen absoluten Größen vorhanden sein. Zur leichteren Handhabung können die unterschiedlichen Größen der Implantate 1, 2, 3, 4 und 5 beispielsweise durch ihre Farbe kenntlich gemacht und dadurch voneinander optisch besser unterschieden werden. Die unterschiedlichen Farben bilden einen Farbcode.

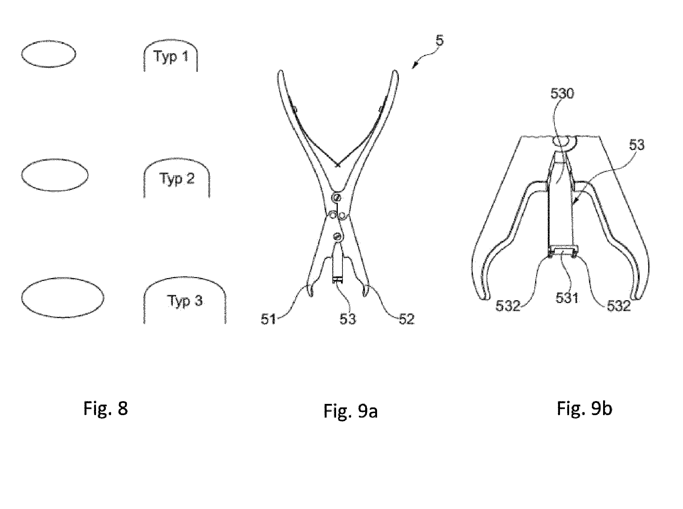

In der Figur 8 ist schematisch dargestellt, welchen Querschnitt die Klammern der Implantate der ersten, zweiten, und dritten Ausführungsform haben können (rechte Seite). Auf der linken Seite sind Ovale aufgezeichnet, die der zum Single-Clip passenden, optimalen Querschnittsfläche des Sternums für die optimale Anpassung entsprechen.

In der Figur 9a ist ein erstes Biegewerkzeug 5 dargestellt, das mit sämtlichen der beschriebenen Implantate 1, 2, 3, 4, 5 und 6 zusammenwirken kann. Das Biegewerkzeug ist zangenförmig mit zwei symmetrischen Backen 51, 52 ausgebildet. Zwischen den Backen 51 und 52 ist ein Niederhalter 53 angeordnet. Dieser ist im Wesentlichen mit einem Drehgelenk der Zange verbunden und wird bei einer Betätigung der Zange so geführt, dass er stets die mittige Position zwischen den Backen 51 und 52 beibehält.

Der Niederhalter 53 (vgl. Figur 9b) weist im Wesentlichen einen Arm 530 auf, an dessen unterem Ende sich senkrecht nach vorne eine Plattform 531 erstreckt. Die Plattform 531 ist rechteckig oder quadratisch ausgebildet. Von der Plattform 531 erstrecken sich nach unten vier Stifte 532, die so angeordnet sind, dass sie in die von den Außenkanten 1000 (vgl. Figur 2) des Verbindungsbereichs 100 des Implantats 1 und den jeweiligen Seitenkanten 1011, 1012, 1021, 1022 der Vorsprünge 111 bzw. 112 gebildeten Winkel eingreifen. Auf diese Weise kann die Plattform 530 sicher am Element 11 zum Eingriff bzw. positionsstabilen Abstützen positioniert werden. Die Plattform 531 kann zumindest bzgl. Bewegungen in ihrer Ebene fixiert bzw. festgelegt werden. Die Plattform 531 und die Stifte 532 bilden ein Positionierelement für das Werkzeug 5.

Die Backen 51 und 52 weisen jeweils Hohlkehlen auf, d.h. die unteren Enden der Backen 51 und 52 weisen jeweils eine zentrale Vertiefung auf, die durch seitliche nach unten hervorstehende Führungsstege begrenzt wird. Auf diese Weise können die Backen 51 und 52 entlang den Klammerelementen, z.B. den Schenkeln 101 bzw. 102, von oben (also vom Verbindungsbereich 100) in Richtung der freien Enden der Schenkel 101 bzw. 102 geführt werden.

Das erste Biegewerkzeug 5 wird als Klammeranlagezange eingesetzt. Zunächst wird das umfassende Klammerelement 100, 101, 102 um das Sternum gelegt. Mithilfe des Biegewerkzeugs 5 werden die Schenkel 101 und 102 der Klammer 10 nach innen aufeinander zu gebogen. Die Klammerenden müssen dabei nicht exakt an die Rückseite des Sternums angeformt werden. Die Klammerenden werden mithilfe des Werkzeugs 5 ca. 45° einwärts gebogen.

Da am Sternum Implantate nicht mittels einer Klemme vor dem Fixieren gehalten werden können und bekannt ist, dass beim Verformen der Klammer 10 diese "abhebt", wird erfindungsgemäß der Niederhalter 53 bereitgestellt, der an dem entsprechenden Element 11 zum Eingriff bzw. zum Abstützen der Implantatkomponente 1 (analog natürlich 2a, 2b, 3a, 3b, 4a) zusammenwirkt. Es versteht sich von selbst, dass die Befestigung der Klammer 10 mithilfe des Werkzeugs 5 an jedem der bisher beschriebenen Implantate 1, 2, 3, 4,5 oder 6 durchgeführt werden kann.

In den Figuren 10a und 10b ist ein weiteres Biegewerkzeug 6 dargestellt, das ebenfalls zangenförmig ausgebildet ist. Die Backen 61 und 62 sind jedoch asymmetrisch ausgebildet. Die längere Backe 61 weist eine geschwungene Struktur auf, wobei das Ende 610 nach innen gebogen ist, um eine Klammer zu umgreifen. Außerdem weist das Ende 610 eine Gleitfläche auf, die entlang der Außenseite der Schenkel 101, 102 (vgl. Figur 1) der in den Implantaten verwendeten Single-Clips entlanggleiten bzw. entlanggeführt werden kann. Um die Führung zu verbessern, können beidseitig der Gleitfläche Führungsvorsprünge vorgesehen sein.

Die kürzere der Backen 62 weist in ihrem Endbereich zwei hervorstehende Stifte/Stege 620 auf, die so ausgebildet und positioniert sind, dass sie an einer Seite des Elements 11 zum Eingriff bzw. Abstützen eines der Single-Clips 1 eingreifen können, während die längere Backe entlang der Außenseite eines der Schenkel 101 bzw. 102 des Single-Clips 1 nach unten in Richtung des freien Endes eines der Schenkel 101 bzw. 102 gleitet und das Ende des Schenkels 101 bzw. 102 im rückseitigen Bereich des Sternums an die Knochenoberfläche anformt. Die längere der Backen wird kreisbogenförmig in Richtung der Klammerenden geführt und formt die Klammerenden, für die Operateure nicht sichtbar, aber spürbar, an das Sternum an.

Die Führungsfläche der längeren der Backen 61 ist vorzugsweise als gerundete Fläche ausgebildet. Die Vertiefung zwischen den Stegen 620 der kürzeren Backe 62 kann, um ein Abrutschen der Zange vom Element 11 zu verhindern, an der Innenseite der Backe abgerundet oder abgeschrägt sein. D.h. die innere Kante der Fläche ist zwischen den Stegen 620 ist abgetragen bzw. abgeschrägt, um bei der Bewegung der längeren Backe 610 nach unten einen sicheren Halt der kürzeren Backe 620 am Element 11 zu gewährleisten

Ein drittes Biegewerkzeug 7, das ebenfalls zangenförmig ausgebildet ist, ist in der Figur 11 dargestellt. Es ist zum Zusammenwirken mit der zweiten und dritten Ausführungsform der Erfindung bestimmt. Während eine erste Backe 71 des Werkzeugs 7 eine im Wesentlichen glatte Innenseite aufweist, weist die zweite Backe 72 einen Vorsprung 720 auf, der komplementär zu den Öffnungen 202 bzw. 302 (vgl. Figuren 3, 4) der Stege 20 bzw. 30 der Implantate 2 bzw. 3 ausgebildet ist. Der Vorsprung 720 greift in eine der Öffnungen 202, 302 ein. Ein entsprechender Vorsprung 720 einer weiteren Zange 7 kann in eine andere Öffnung 202, 302 desselben Implantats 2 bzw. 3 eingreifen. Anschließend kann eine der Zangen 7 betätigt, z.B. gegenüber der anderen Zange 7 verdreht werden. Die Zangen 7 greifen so sicher am Steg 20 bzw. 30 an, dass dieser mithilfe der Werkzeuge 7 definiert verbogen werden kann, beispielsweise entlang der Achse A gedreht (torquiert) werden kann. Aufgrund der nicht runden Form der Öffnungen 202 (in etwa quadratische oder rautenförmige Form) in den Stegen 20, 30 kann der Steg 20, 30 auch in beide Richtungen in der Ebene, die vom Steg aufgespannt wird, gebogen werden, oder der Steg 20, 30 kann in der durch sich entlang der Achse A erstreckenden Symmetrieebene nach oben bzw. nach unten verbogen werden.

In einer nicht dargestellte Ausführungsform kann die Backe 72 an der Innenseite des zangenartigen Werkzeugs 7 im Wesentlichen flach/eben ausgeführt sein, wobei der Vorsprung 720 von dieser ebenen Oberfläche hervorragt, d.h. der Vorsprung erstreckt sich von der ebenen Oberfläche der Innenseite der Backe 72, sodass die Oberfläche des Vorsprungs 720 nicht bündig mit der der Innenseite der Backe 72 ist. Seitlich schließt bei dieser nicht dargestellten Ausführungsform die I Backe 72 in etwa mit der jeweiligen Ecke des Vorsprungs 720 ab.

Die Figuren 12a und 12b zeigen ein viertes Biegewerkzeug 8, das in Form einer Zange mit zwei Backen 81 und 82 ausgebildet ist. Die Backen 81 und 82 weisen jeweils eine Vorderseite auf, die an einer Innenkante 810 bzw. 820 endet. Im Bereich der Innenkanten 810 und 820 ist eine Vertiefung 811 bzw. 821 ausgebildet, die sich von der Kante ausgehend nach außen erstreckt und eine Außenkontur 8110 bzw. 8210 aufweist, die gekrümmt bzw. nach außen hin ausgebuchtet sein kann. Die Figur 12c zeigt das Biegewerkzeug von vorne, wobei die Außenkonturen 8110 und 8120 der Vertiefungen 811 bzw. 821 konkav ausgebildet sind, insbesondere so, dass sie rautenförmige Rahmenabschnitte 201 formschlüssig aufnehmen können. Die Aussparungen 811 und 821 können mit einer an die Raute des Implantatstegs angepasste Knntur ausgebildet sein. Die Krümmung bzw. Ausbuchtung ist so angepasst, dass die Vertiefungen 811 und 821 gemeinsam ein Stegelement 200, 300 aufnehmen können. Durch Betätigung des Werkzeugs 8 greifen die Hinterwände 8110 und 8120 der Vertiefungen 811 bzw. 821 an den Rahmen 201 des Stegelements 200, 300 an. Die gegenüberliegenden Steghälften können durch weitere Betätigungen verformt werden, so dass die Öffnung 202, 302 in eine Raute verformt wird, deren längere Diagonale entlang der Achse A liegt. Auf diese Weise wird der Steg 20 bzw. 30 verlängert. Das Werkzeug aus Figuren 12a und 12b ist zur Verbindung von Implantatkomponenten gemäß Figur 5 und Figur 6 erforderlich, zur Stegverlängerung ist bei der beschriebenen Zange gemäß Figur 12c ein Maulende mit einer rautenförmigen Führung vorgesehen.

Es ist der Einsatz weiterer Biegewerkzeuge denkbar, die entweder an den Elementen 11 zum Eingriff bzw. zum Angreifen bzw. zum Abstützen eines Single-Clips 1, 2a, 2b, 3a, 3b, 4a, 5a, durch Umgreifen von Rahmenabschnitten 201, 301 des Rahmens 201 der Stegelemente 200, 300 bzw. durch Eingreifen in die vom Rahmen 201, 301 begrenzte Öffnung 202, 302 eines Stegelements 200, 300 am Implantat definiert positioniert und festgelegt werden, oder das Implantat 1, 2, 3, 4, 5 greifen können, insbesondere formschlüssig, sodass Biegebewegungen auf einzelne Komponenten bzw. Teile des Implantats 1, 2, 3, 4, 5 übertragen werden können. Dadurch kann eine Bearbeitung und Verformung des Implantats mit möglichst großer Zuverlässigkeit und in möglichst kurzer Zeit durchgeführt werden.

So ist beispielsweise ein Biegewerkzeug vorstellbar, das einen Steg 20, 30 in der zweiten und dritten Ausführungsform 2 bzw. 3 der Erfindung stauchen kann. Dazu kann an beiden Backen eines zangenartigen Instruments jeweils ein Vorsprung ausgebildet sein, der der Kontur der Öffnungen 202, 302 der Stegelemente 200, 300 entspricht. Diese Vorsprünge können in Öffnungen 202, 203 der beiden äußeren Stegelemente 200, 300 eingreifen. Durch Betätigung der Zange werden die Backen zueinander geführt, wodurch es zu einer Stauchung des mittleren Stegelements 200, 300 kommt, sodass die Öffnung 202, 302 des mittleren Stegelements 200, 300 in eine Raute verformt wird, deren längere Diagonale senkrecht zur Achse A liegt. Auf diese Weise wird der Steg 20, 30 insgesamt verkürzt.

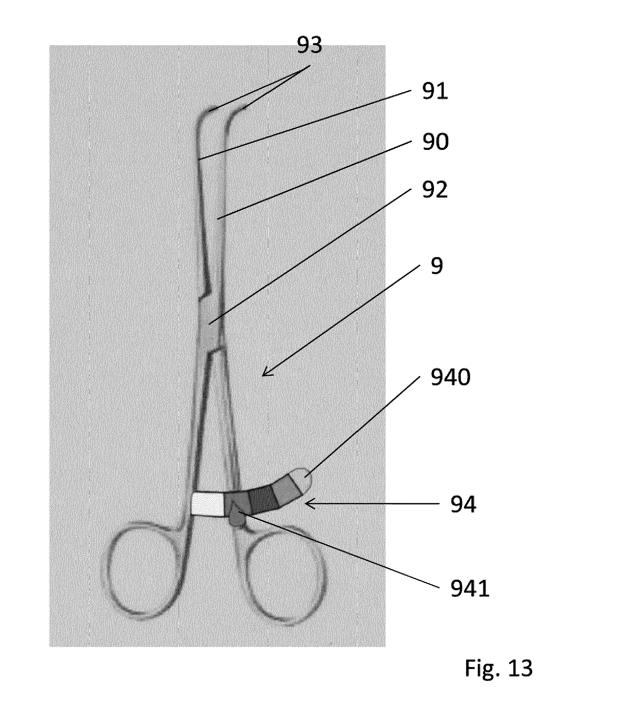

In der Figur 13 ist ein erfindungsgemäßes Implantat-Auswahlinstrument 9 dargestellt. Dieses weist gegeneinander bewegliche Hebel 90, 91 auf, die um eine Achse 92 drehbar zueinander gelagert sind.

Das Instrument 9 soll zwei unterschiedliche Aufgaben erfüllen: Zum einen soll das Instrument 9 dazu dienen, sternumnahe Perforationen der Weichteile zur Aufnahme der Klammerenden stumpf zu präparieren. Stumpfe Präparation ist wichtig, damit keine Blutgefäße verletzt werden. Das Maul 93 ist nach unten hin abgebogen, sodass, unabhängig von der Eindringtiefe der gewinkelten Maulteile, ein konkretes "Messergebnis" auf die Skala 94 übertragen wird. Die Maulenden tauchen parallel zu den Außenseiten des Sternums ein und übertragen somit die Sternumbreite auf die Skala 940.So ist gewährleistet, dass ein senkrechtes, paralleles Eindringen in die Weichteile links und rechts des Brustbeins durchgeführt werden kann, weshalb das Instrument in jedem Fall abgebogen und nicht gerade sein muss. Bei diesem Vorgang sollen Löcher entstehen, die die Implantatenden gut aufnehmen können, keinesfalls nur nadelstichartige Perforationen.

Die zweite Aufgabe des Instruments besteht darin, über eine Skala 940 das jeweils passende Implantat hinsichtlich der Breite der Klammern zu ermitteln. Die Messeinrichtung 94 weist eine farblich abgestufte Skala 940 auf, die etwa ein gebogener und dünner Metallstreifen ist, der umseitig analog den farblich bzgl. ihrer Größe definierten Implantattypen dieselben Farben aufweist. Die Skala ist an einem der beiden Hebel 91 befestigt. Dies bedeutet, dass, wenn der Anzeiger 941 (der am anderen Hebel 90 befestigt ist) bei einer Messung des Abstands zwischen den Maulenden 93 auf ein bestimmtes Feld der Skala 940 zeigt, ein diesem Feld entsprechend großes Implantat eingesetzt werden soll. Die Messeinrichtung 94 ist als eine Art Messzirkel ausgebildet.

Bei der Herstellung der Skala 940 mit den unterschiedlichen Farbfeldern muss darauf geachtet werden, dass diese resistent gegenüber Verschleiß ist und sich die Farben nicht verändern, da das Instrument bei jedem Eingriff zwingend verwendet werden muss und auch hinsichtlich der Reinigungs- und Sterilisationsverfahren hohem Verschleiß ausgesetzt ist. Die Skala 940 solle daher aus einem belastbaren Material bestehen. Ein geeignetes Material wäre Titan, da dieses Material anodisierfähig ist. Denkbar wäre auch eine Skala aus anderen belastbaren, resistenten Materialien, die dauerhaft farblich kenntlich gemacht werden können, beispielsweise durch eine Skala mit entsprechend verschleißfesten Farben. Jedenfalls wird die Skala analog zu verschiedenen Implantattypen und -größen ausgerichtet.

Um die Messung möglichst genau durchführen zu können, müssen die Maulteile 93 relativ steif ausgebildet sein, sodass nicht durch zu hohe Flexibilität die Messergebnisse an der Skala beeinträchtiget werden. Außerdem können bei ausreichender Steifigkeit der Maulteile die Weichteile mit gewissem Druck perforiert werden.

Die Ermittlung der korrekten Implantatgröße erfolgt durch Umgreifen des Rippenabschnitts, an dem das Implantat angebracht werden soll, durch die Enden der Hebel, also durch die beiden Hälften des Maulteils 93, und anschließendes Ablesen der richtigen Größe des Implantats an der Skala 940.

## Patentansprüche

1. Implantatkomponente (1, 2a, 2b, 3a, 3b, 4a) zur Sternum-Osteosynthese und Biegewerkzeug (5, 6) zur Befestigung der Implantatkomponente am Sternum, wobei die Implantatkomponente eine Klammer (10) mit einem ersten Schenkel (101) und einem zweiten Schenkel (102) umfasst, wobei der erste Schenkel (101) und der zweite Schenkel (102) über einen Verbindungsbereich (100), der beidseitig jeweils eine Kante (1000) aufweist, verbunden sind,
und das Biegewerkzeug (5, 6) zwei Klemmbacken (51, 52, 61, 62) und ein Positionierelement (531, 532, 620) zum Eingriff mit der Implantatkomponente (1, 2a, 2b, 3a, 3b, 4a) oder zum Abstützen des Biegewerkzeugs (5, 6) an der Implantatkomponente (1, 2a, 2b, 3a, 3b, 4a) derart umfasst, dass mittels Betätigung wenigstens einer der Klemmbacken ein Schenkel der Klammer nach innen unter das Sternum biegbar ist.
wobei
das Positionierelement (531, 532, 620) zum Eingriff oder zum Abstützen des Biegewerkzeugs (5, 6) an der Implantatkomponente (1, 2a, 2b, 3a, 3b, 4a) wenigstens zwei Vorsprünge (532, 620) aufweist, und die Implantatkomponente (1, 2a, 2b, 3a, 3b, 4a) am Verbindungsbereich (100) ein Element (11) zum gegenseitigen Eingriff mit bzw. zum positionsstabilen Abstützen eines kompatiblen Positionierelements eines Biegewerkzeugs (5, 6) aufweist, wobei das Element (11) einen ersten Vorsprung (111) aufweist, der sich von einer ersten Seite des Verbindungsbereichs (100) seitlich erstreckt, und einen zweiten Vorsprung (112), der sich von der gegenüberliegenden Seite des Verbindungsbereichs (100) seitlich erstreckt, wobei die Seitenkanten (1111, 1112, 1121, 1122) der Vorsprünge (111, 112) mit der jeweiligen Kante (1000) des Verbindungsbereichs (100) einen Winkel zum Eingriff der wenigstens zwei Vorsprünge des Positionierelements des Biegewerkzeugs bilden.

2. Implantatkomponente (1, 2a, 2b, 3a, 3b, 4a) und Biegewerkzeug nach Anspruch 1, **dadurch gekennzeichnet, dass**
der erste Vorsprung (111) und/oder der zweite Vorsprung (112) eine erste Stirnkante (1110) bzw. eine zweite Stirnkante (1120) und zwei Seitenkanten (1111, 1112, 1121, 1122) aufweisen.

3. Implantatkomponente (1, 2a, 2b, 3a, 3b, 4a) und Biegewerkzeus nach Anspruch 1 oder 2,
**dadurch gekennzeichnet, dass**
die Seitenkanten (1111, 1112, 1121, 1122) mit der jeweiligen Kante (1000) des Verbindungsbereichs (100) einen spitzen Winkel (α) einschließen.

4. Implantatkomponente (1, 2a, 2b, 3a, 3b, 4a) und Biegewerkzeug nach Anspruch 3,
**dadurch gekennzeichnet, dass**
der Winkel (α) größer als 60°, insbesondere größer als 80°, und kleiner als 90°, insbesondere kleiner als 85° ist.

5. Implantatkomponente (1, 2a, 2b, 3a, 3b, 4a) und Biegewerkzeug nach einem der vorgehenden Ansprüche,
**dadurch gekennzeichnet, dass**
die erste Stirnkante (1110) und/oder die zweite Stirnkante (1120) gekrümmt ausgebildet ist.

6. Implantatkomponente (1, 2a, 2b, 3a, 3b, 4a) und Biegewerkzeug nach einem der vorgehenden Ansprüche,
**dadurch gekennzeichnet, dass**
die Oberfläche des Elements (11) zum Eingriff bzw. Abstützen bündig mit dem Verbindungsbereich (100) der Klammer (10) ausgebildet ist.

7. Implantatkomponente (1, 2a, 2b, 3a, 3b, 4a) und Biegewerkzeug nach einem der vorgehenden Ansprüche,
**dadurch gekennzeichnet, dass**
der erste Vorsprung (111) und/oder der zweite Vorsprung (112) dieselbe Dicke aufweisen wie der Verbindungsbereich (100) des Implantats (1).

8. Implantatkomponente (1, 2a, 2b, 3a, 3b, 4a) und Biegewerkzeug nach einem der vorgehenden Ansprüche,
**dadurch gekennzeichnet, dass**
das Positionierelement (531, 532) des Biegewerkzeugs als Niederhalter zum Anpressen des Implantats (1, 2, 3, 4) an das Sternum während des durch zwei Klemmenbacken (51, 52) ausgeübten Klemmvorgangs ausgebildet ist.

9. Implantatkomponente (1, 2a, 2b, 3a, 3b, 4a) und Biegewerkzeug (6) nach einem der vorgehenden Ansprüche,
**dadurch gekennzeichnet, dass**
das Positionierelement des Biegewerkzeugs zwei hervorstehende Stifte (620) aufweist, die an einer der Klemmbacken (62) angeordnet sind.

10. Implantatsystem (4) zur Sternum-Osteosynthese, umfassend:
wenigstens eine Implantatkomponente (4a) und Biegewerkzeug nach einem der Ansprüche 1 bis 7,
wobei die Implantatkoponente eine Befestigungseinrichtung (42) zur Befestigung eines stabförmigen Verbindungselements (44) an der Befestigungseinrichtung (42) aufweist, wobei die Befestigungseinrichtung (42) am Element (41) zum Eingriff bzw. zum positionsstabilen Abstützen befestigt ist.

11. Implantatsystem (4) nach Anspruch 10,
**dadurch gekennzeichnet, dass**
die Befestigungseinrichtung (42) einen Grundkörper (420) mit einer Auflagefläche und seitlich der Auflagefläche angeordnete Führungselemente (43) aufweist, wobei die Führungselemente (43) jeweils einen Steg und einen sich von der Oberkante des Stegs nach innen erstreckenden Führungsvorsprung aufweisen, die mit einem Seitenbereich der Auflagefläche jeweils im Querschnitt U-förmige Führungsaufnahmen begrenzen.

12. Implantatsystem (4) nach einem der Ansprüche 10 bis 11
**dadurch gekennzeichnet, dass**
die Führungsvorsprünge der Führungselemente (43) eine Eingriffsstruktur, insbesondere eine Zahnstruktur, aufweisen.

## Claims

1. Implant component (1, 2a, 2b, 3a, 3b, 4a) for sternal osteosynthesis and bending tool (5, 6) for the purpose of attaching at the sternum, wherein the implant component comprising a clamp (10) having a first prong (101) and a second prong (102), wherein the first prong (101) and the second prong (102) are connected by a connecting portion (100), having on both sides a side edge (1000) respectively, and said bending tool (5, 6) comprising two clamping jaws (51, 52, 61, 62) and a positioning member (531, 532, 620) for the purpose of providing engagement with the implant component (1, 2a, 2b, 3a, 3b, 4a) or for the purpose of providing support for the bending tool (5, 6) at the implant component (1, 2a, 2b, 3a, 3b, 4a), wherein upon actuation of at least one of the clamping jaws one prong of the clamp is used to bend inwards under the sternum, wherein the positioning member (531, 532, 620) comprising for the purpose of providing engagement with or support for the bending tool at the implant component (1, 2a, 2b, 3a, 3b, 4a) at least two protrusions (532, 620) and the implant component (1, 2a, 2b, 3a, 3b, 4a) comprising at the connecting portion (100) a member (11) for the purpose of providing mutual engagement with or positionally stable support for a compatible positioning member of a bending tool (5, 6) wherein the member (11) comprises a first protrusion (111), which extends laterally from the first side of the connecting portion (100), and a second protrusion (112), which extends laterally from the opposite side of the connecting portion (100), wherein the side edges (1111, 1112, 1121, 1122) of the protrusions (111, 112) forming an angle with the respective edge (1000) of the connecting portion (100) for the purpose of engagement with at least two protrusions of the positioning member of the bending tool.

2. Implant component (1, 2a, 2b, 3a, 3b, 4a) and bending tool in accordance with claim 1,
**characterized by** the fact that
the first protrusion (111) and/or the second protrusion (112) have a first end edge (1110) and/or a second end edge (1120) and two side edges (1111, 1112, 1121, 1122).

3. Implant component (1, 2a, 2b, 3a, 3b, 4a) and bending tool in accordance with claim 1 or 2,
**characterized by** the fact that
the side edges (1111, 1112, 1121, 1122) form an acute angle (α) with the respective edge (1000) of the connecting portion (100).

4. Implant component (1, 2a, 2b, 3a, 3b, 4a) in accordance with claim 3,
**characterized by** the fact that
the angle (α) is greater than 60°, in particular greater than 80°, and smaller than 90°, in particular smaller than 85°.

5. Implant component (1, 2a, 2b, 3a, 3b, 4a) and bending tool in accordance with any of the previous claims,
**characterized by** the fact that
the first end edge (1110) and/or the second end edge (1120) is formed so as to be curved.

6. Implant component (1, 2a, 2b, 3a, 3b, 4a) and bending tool in accordance with any of the previous claims,
**characterized by** the fact that
the surface of the member (11) for providing engagement or support is flush with the connecting portion (100) of the clamp (10).

7. Implant component (1, 2a, 2b, 3a, 3b, 4a) and bending tool in accordance with any of the previous claims,
**characterized by** the fact that
the first protrusion (111) and/or the second protrusion (112) have the same thickness (100) as the connecting portion of the implant (1).

8. Implant component (1, 2a, 2b, 3a, 3b, 4a) and bending tool in accordance with any of the previous claims,
**characterized by** the fact that
the positioning member (531, 532) be formed as a hold-down device for the purpose of pressing the implant (1, 2, 3, 4) against the sternum during the clamping process performed by the two clamping jaws (51, 52).

9. Implant component (1, 2a, 2b, 3a, 3b, 4a) and bending tool (6) in accordance with any of the previous claims,
**characterized by** the fact that
the positioning member of the bending tool has two protruding pins (620) arranged at one of the clamping jaws (62).

10. Implant system (4) for sternal osteosynthesis, comprising:
at least one implant component (4a) and bending tool in accordance with any of claims 1 to 7,
wherein the implant component comprising an attaching device (42) for attaching a rod-shaped connecting member (44) at the attaching device (42), with the attaching device (42) attached at the member (41) for the purpose of providing engagement and/or positionally stable support.

11. Implant system (4) in accordance with claim 10,
**characterized by** the fact that
the attaching device (42) has a base (420) with a contact surface and guide members (43) arranged laterally thereto, wherein the guide members (43) each have a fillet and a guide protrusion that extends inwardly from the fillet's upper edge to form guide receptacles with one side portion of the contact surface, said receptacles having a U-shaped cross-section.

12. Implant system (4) in accordance with any of claims 10 to 11,
**characterized by** the fact that
the guide protrusions of the guide members (43) have an engagement structure, partiularly a toothed structure.

## Revendications

1. Composant d'implant (1, 2a, 2b, 3a, 3b, 4a) pour l'ostéosynthèse du sternum et outil de cintrage (5, 6) pour la fixation du composant d'implant sur le sternum, cependant que le composant d'implant comprend une agrafe (10) avec un premier montant (101) et un second montant (102), cependant que le premier montant (101) et le second montant (102) sont reliés par une zone de liaison (100) qui présente respectivement une arête (1000) des deux côtés et l'outil de cintrage (5, 6) comprend deux mâchoires de serrage (51, 52, 61, 62) et un élément de positionnement (531, 532, 620) pour l'engrènement avec le composant d'implant (1, 2a, 2b, 3a, 3b, 4a) ou pour l'appui de l'outil de cintrage (5, 6) sur le composant d'implant (1, 2a, 2b, 3a, 3b, 4a) de telle manière qu'un montant de l'agrafe peut être courbé vers l'intérieur sous le sternum par l'actionnement d'au moins l'une des mâchoires de serrage, cependant que l'élément de positionnement (531, 532, 620) présente au moins deux saillies (532, 620) pour l'engrènement ou pour l'appui de l'outil de cintrage (5, 6) sur le composant d'implant (1, 2a, 2b, 3a, 3b, 4a) et le composant d'implant (1, 2a, 2b, 3a, 3b, 4a) présente, sur la zone de liaison (100), un élément (11) pour l'engrènement mutuel avec ou pour l'appui en position stable d'un élément de positionnement compatible d'un outil de cintrage (5, 6), cependant que l'élément (11) présente une première saillie (111) qui s'étend latéralement à partir d'un premier côté de la zone de liaison (100) et une seconde saillie (112) qui s'étend latéralement à partir du côté opposé de la zone de liaison (100), cependant que les arêtes latérales (1111, 1112, 1121, 1122) des saillies (111, 112) forment, avec l'arête respective (1000) de la zone de liaison (100) un angle pour l'engrènement des deux saillies qui existent au moins de l'élément de positionnement de l'outil de cintrage.

2. Composant d'implant (1, 2a, 2b, 3a, 3b, 4a) et outil de cintrage selon la revendication 1,
**caractérisé en ce que**
la première saillie (111) et/ou la seconde saillie (112) présente une première arête frontale (1110) ou une seconde arête frontale (1120) et deux arêtes latérales (1111, 1112, 1121, 1122).

3. Composant d'implant (1, 2a, 2b, 3a, 3b, 4a) et outil de cintrage selon la revendication 1 ou 2,
**caractérisé en ce que**
les arêtes latérales (1111, 1112, 1121, 1122) incluent un angle aigu (α) avec l'arête respective (1000) de la zone de liaison (100).

4. Composant d'implant (1, 2a, 2b, 3a, 3b, 4a) et outil de cintrage selon la revendication 3,
**caractérisé en ce que**
l'angle (α) est plus grand que 60°, en particulier plus grand que 80° et plus petit que 90°, en particulier plus petit que 85°.

5. Composant d'implant (1, 2a, 2b, 3a, 3b, 4a) et outil de cintrage selon l'une des revendications précédentes,
**caractérisé en ce que**
la première arête frontale (1110) et/ou la seconde arête frontale (1120) est configurée courbée.

6. Composant d'implant (1, 2a, 2b, 3a, 3b, 4a) et outil de cintrage selon l'une des revendications précédentes,
**caractérisé en ce que**
la surface de l'élément pour l'engrènement ou l'appui est configurée à fleur avec la zone de liaison (100) de l'agrafe (10).

7. Composant d'implant (1, 2a, 2b, 3a, 3b, 4a) et outil de cintrage selon l'une des revendications précédentes,
**caractérisé en ce que**
la première saillie (111) et/ou la seconde saillie (112) présente la même épaisseur que la zone de liaison (100) de l'implant (1).

8. Composant d'implant (1, 2a, 2b, 3a, 3b, 4a) et outil de cintrage selon l'une des revendications précédentes,
**caractérisé en ce que**
l'élément de positionnement (531, 532) de l'outil de cintrage est configuré comme un élément de maintien vers le bas pour presser l'implant (1, 2, 3, 4) contre le sternum pendant l'opération de serrage exercée par deux mâchoires de serrage (51, 52).

9. Composant d'implant (1, 2a, 2b, 3a, 3b, 4a) et outil de cintrage selon l'une des revendications précédentes,
**caractérisé en ce que**
l'élément de positionnement (531, 532) de l'outil de cintrage présente deux goujons qui font saillie (620) qui sont placés sur l'une des mâchoires de serrage (62).

10. Système d'implant (4) pour l'ostéosynthèse du sternum comprenant : au moins un composant d'implant (4a) et un outil de cintrage selon l'une des revendications 1 à 7, cependant que le composant d'implant présente un dispositif de fixation (42) pour la fixation d'un élément de liaison en forme de barre (44) sur le dispositif de fixation (42), cependant que le dispositif de fixation (42) est fixé à l'élément (41) pour l'engrènement ou pour l'appui en position stable.

11. Système d'implant (4) selon la revendication 10,
**caractérisé en ce que**
le dispositif de fixation (42) présente un corps de base (420) avec une surface d'appui et des éléments de guidage (43) placés latéralement de la surface d'appui, cependant que les éléments de guidage (43) présentent respectivement une barrette et une saillie de guidage qui s'étend vers l'intérieur à partir de l'arête supérieure de la barrette qui délimitent respectivement des logements de guidage de section en forme de U avec une zone latérale de la surface d'appui.

12. Système d'implant (4) selon l'une des revendications 10 à 11,
**caractérisé en ce que**
les saillies de guidage des éléments de guidage (43) présentent une structure d'engrènement, en particulier une structure de dents.
